# EUROPÄISCHE PATENTSCHRIFT

(11) **EP 0 747 034 B1**
(45) Veröffentlichungstag und Bekanntmachung des Hinweises auf die Patenterteilung: **16.08.2001**
(21) Anmeldenummer: 96108638.6
(22) Anmeldetag: 30.05.1996
(51) Int. Cl.: A61K 6/06

(54) **Pulvermischung zur Herstellung eines Opakers in Pastenform**
Powder mixture for the preparation of a paste of opaque material
Mélange de poudres pour la préparation d'une pâte de matériau opaque

(30) Priorität: 08.06.1995 EP 95108803
(43) Veröffentlichungstag der Anmeldung: 11.12.1996
(73) Patentinhaber: Vita Zahnfabrik H. Rauter GmbH & Co. KG, D-79704 Bad Säckingen (DE)
(72) Erfinder: Thiel, Norbert, Dr., 79713 Bad Säckingen (DE); Herbst, Kerstin, 79664 Wehr (DE)
(74) Vertreter: Meyers, Hans-Wilhelm

(56) Entgegenhaltungen:
- EP-A- 0 125 036
- EP-A- 0 280 985
- EP-A- 0 332 887
- EP-A- 0 518 454
- US-A- 5 030 097
- US-A- 5 176 747

## Beschreibung

Die vorliegende Erfindung betrifft eine Pulvermischung zur Herstellung eines Opakers und eine Pulvermischung in Pastenform.

Zahnersatz, wie Zahnkronen und -brücken, wird häufig aus einem Metallgerüst angefertigt, welches mit unterschiedlichen Materialien verblendet wird. Weit verbreitet sind dabei mit Keramik verblendete Restaurationen, die medizinischen und ästhetischen Bedürfnissen in hohem Maße Rechnung tragen. Dabei ist ein mehrschichtiger Aufbau des Verblendmaterials auf dem metallischen Untergrund bekannt. Als erste Schicht wird häufig eine sogenannte Opakerschicht auf das Metallgerüst der Restauration aufgetragen, um die Metallfarbe des Unterbaus abzudecken.

Aus der EP 0 518 454 A2 und den darin genannten den Stand der Technik bildenden Druckschriften geht beispielsweise hervor, daß bei Verwendung von größeren Mengen Zirkondioxid als Trübungsmittel zu Glaspulvern Mischungen erhalten werden, die sich als Opaker geeignet erweisen. Die dort vorgestellten Opaker bestehen aus dem Glaspulver als Grundmischung sowie dem in größerem Maße zugemischten Zirkonoxid als Trübungsmittel. Als wesentlich wird in der EP 0 518 454 beschrieben, daß 10 bis 60 Gew.-% Zirkondioxid als Trübungsmittel vorhanden sein müssen. Gegebenenfalls kann die Mischung mit Pigmenten versehen sein, um bereits in der ersten Schicht einen der späteren Zahnfarbe entsprechenden Farbton zu erzielen oder sich diesem anzunähern.

Nachteilig an dem dort genannten System ist insbesondere die hohe Brenntemperatur von 980°C. Bei diesen Temperaturen ist die Warmfestigkeit einiger Dentallegierungen nicht mehr gegeben, so daß sich beim Aufbrennen der Verblendkeramik das Metallgerüst verzieht und damit die Paßgenauigkeit der prothetischen Arbeit nicht mehr gegeben ist.

In der EP 0 280 985 ist ebenfalls eine opake Dentalkeramik beschrieben. Bei diesem System wirkt sich die Korngröße von kleiner 40 *µ*m nachteilig auf die Auftrageigenschaften des Opakers aus. Ebenfalls nachteilig an dem dort beschriebenen System ist, daß die Oberfläche nach dem Auftrag des Opakers mit Streukristallen bestreut werden muß, um eine gute Deckkraft und eine gleichmäßige Oberfläche ohne Risse zu erhalten.

Die US-PS 5,176,747 offenbart Porzellanzusammensetzung, die zur Verwendung als Porzellanschichten auf Metallbasen für Zahnersatz geeignet sind und eine Fusionstemperatur von ungefähr 800°C und geringer aufweist. Die Zusammensetzungen können als Überzüge auf Zahnersatz aus Titan oder Titanlegierungen verwendet werden, da die thermischen Expansionskoeffizienten nahe derjenigen von Titan und seinen Legierungen liegt. Die beschriebenen Zusammensetzungen weisen bis zu 3 Gew.-% Ceroxid auf.

Die EP-A-0 332 887 betrifft ein Verfahren zur Herstellung von metallkeramischen Dentalrestaurationen. Zur Erzielung einer optimalen ästhetischen Anpassung der Dentalrestauration an das Restgebiß werden individuelle Charakteristika des natürlichen, zu ersetzenden Zahnes bzw. der natürlichen benachbarten Zähne im Zuge des Aufbaus der metallkeramischen Dentalrestauration durch gezieltes Einlegen oder Zumischen einer keramischen Malfarbe zu der Dentinmasse und/oder Schmelzmasse nachgebildet. Bei Anwendung des beschriebenen Verfahrens kann die Anzahl der erforderlichen unterschiedlich pigmentierten und getrübten Keramikmassen drastisch reduziert werden. Die Mischung enthält als Trübungsmittel in der Opakermasse 10 bis 20 % der Oxide Zinnoxid, Ceroxid und Zirkonsilikat.

Die US-A-5,030,097 betrifft eine Mischung vorzugsweise als kolloidale Suspension feinverteilter Teilchen einer Selten Erde Metalloxidfraktion wie Ceroxid in einer Flüssigkeit. Diese Flüssigkeit kann gänzlich aus Wasser bestehen aber ebenso andere Substanzen beinhalten wie Essigsäure, 1,3-Butandiol oder Methanol. Die Mischung wird als Benetzungs- und Suspendierungsmittel für einen Opaker verwendet, um in erster Linie Verfärbungsprobleme von Dentalkeramikmassen zu lösen, wenn diese im Zusammenhang mit silberhaltigen Dentallegierungen verwendet werden.

Das technische Problem der vorliegenden Erfindung besteht darin, die genannten Nachteile zu vermeiden und einen Opaker bereitzustellen, der einfach und preiswert herstellbar ist und sich durch sehr gute Auftrageigenschaften, niedrige Brenntemperaturen, sehr gute Deckkraft und einen guten Haftverbund zum Metall auszeichnen soll. Außerdem sollen keine Verfärbungen im Übergangsbereich Keramik-Metall bzw. an den Rändern der Restauration auftreten.

Überraschenderweise wird das der Erfindung zugrunde liegende Problem gelöst durch einen Opaker, der auf einer Pulvermischung, enthaltend als Komponenten eine oder mehrere Feldspatfritten, CeO₂ als Trübungsmittel, gegebenenfalls weitere Trübungsmittel sowie gegebenenfalls Pigmente, basiert. Die Feldspatfritte besteht aus mehreren Feldspatfrittenkomponenten verschiedener chemischer Zusammensetzung. Durch die chemische Zusammensetzung werden die physikalischen Eigenschaften der Feldspatfritte wie beispielsweise Brenntemperatur, Wärmeausdehnung, Transformationspunkt und Erweichungstemperatur beeinflußt. So können beispielsweise bei Verwendung verschiedener Feldspatfritten diese Eigenschaften des herzustellenden Opakers gezielt variiert werden.

Als vorteilhaft hat sich erwiesen, daß die Feldspatfritten eine maximale Korngröße von unter 20 *µ*m aufweisen. Es hat sich herausgestellt, daß größere Körnungen die Verarbeitungseigenschaften verschlechtern.

Zu der Frittenmischung werden als Trübungsmittel neben CeO₂ insbesondere Metalloxide wie SnO₂, TiO₂, ZrO₂ und ähnliche eingesetzt. Die als Trübungsmittel eingesetzten Oxide weisen eine eine mittlere Korngröße von d50 < 1 *µ*m und eine maximale Korngröße < 2 *µ*m sowie eine spezifische Oberfläche von 1 bis 15 m²/g auf und sollen eine Reinheit >99% aufweisen.

Oxide mit anderer Kornverteilungscharakteristik und/oder einer größeren spezifischen Oberfläche sind als Trübungsmittel in dem aus der Pulvermischung herzustellenden Pastenopaker nicht geeignet, da Kornverteilung und spezifische Oberfläche die Auftrageigenschaften unmittelbar beeinflussen. Gegebenenfalls werden der weißen Pulvermischung Farbpigmente zugesetzt, um zahnfarbene Restaurationen zu erhalten. Als Pigmente werden die in der Zahntechnik üblichen Farbpigmente zugesetzt.

Vorzugsweise weisen die Hauptkomponenten der erfindungsgemäßen Pulvermischung folgende Mengenbereiche der Zusammensetzung auf:
- 30 bis 65 Gew.-% SiO₂
- 2 bis 6 Gew.-% Na₂O
- 3 bis 15 Gew.-% K₂O
- 5 bis 15 Gew.-% Al₂O₃
- 0,5 bis 10 Gew.-% CaO
- 0,5 bis 10 Gew.-% B₂O₃
- 0,5 bis 50 Gew.-% CeO₂.

Insbesondere bevorzugt sind 35 bis 45 Gew.-% SiO₂, 4 bis 5 Gew.-% Na₂O, 7 bis 9 Gew.-% K₂O, 10 bis 12 Gew.-% Al₂O₃, 2 bis 3 Gew.-% CaO sowie 1 bis 3 Gew.-% B₂O₃.

Gegebenenfalls kann die Pulvermischung weitere Komponenten enthalten, wobei bis zu 9 Gew.-% ZrO₂, 50 Gew.-% SnO₂ sowie 50 Gew.-% TiO₂ in der Mischung enthalten sind. Bevorzugt sind Gehalte an SnO₂, CeO₂ und/oder TiO₂ von 10 bis 30 Gew.-% und/oder Gehalte an ZrO₂ von 3 bis 9 Gew.-%.

Die erfindungsgemäße Pulvermischung wird mit Flüssigkeiten, die einzeln oder als Gemisch eingesetzt werden können, zu einer Mischung in Pastenform verarbeitet. Die erfindungsgemäße Mischung kann zusätzlich gegebenenfalls Hilfsstoffe enthalten. Die Zugabe der Flüssigkeiten wird so eingestellt, daß sich eine homogene, weiche, leicht mit einem Pinsel zu applizierende Paste mit einer Konsistenz ähnlich der einer normalen Creme ergibt. Als Flüssigkeiten kommen insbesondere Wasser (entmineralisiertes oder destilliertes Wasser), für Verblendkeramik handelsübliche Modellierflüssigkeiten (z.B. Vita Omega Opaque Liquid, Vita Omega 800 Opaque Liquid), Glycerin, zweiwertige Alkohole, Polyethylenglycole und/oder Silikonöle in Betracht. Als bevorzugt einsetzbar haben sich Mischungen von 1 bis 3 Teilen destilliertem Wasser, 15 bis 21 Teilen eines dreiwertigen Alkohols wie Glycerin und 75 bis 85 Teilen eines zweiwertigen Alkohols wie Butandiol-1.3 erwiesen. Auch eine Mischung von 90 bis 100 Teilen Butandiol-1.3 und 2 bis 7 Teilen Silikonöl, wie Baysilon® -Öl M100, oder eine Mischung aus 40 bis 75 Teilen Butandiol-1.3, 5 bis 12 Teilen Polyethylenglycol wie PEG 400, 20 bis 30 Teilen Gly-cerin und 0,5 bis 5 Teilen deionisiertem Wasser haben sich als geeignet erwiesen.

In der erfindungsgemäßen Mischung in Pastenform (Pastenopaker) sind die Abmischungsverhältnisse von Flüssigkeit(en) zu Pulvermischung vorzugsweise zwischen 1 : 4 bis 1 : 1. Als insbesondere vorteilhaft hat sich eine Mischung bestehend aus einem Verhältnis Pulveropaker zur Flüssigkeit von 7 : 3 erwiesen.

Als Hilfsstoffe kommen insbesondere Mittel zur Verhinderung der Entmischung von Pulvermischung und Flüssigkeit (Stabilisatoren) sowie Konservierungsstoffe in Betracht.

Um Entmischungen, d. h. Trennung der festen von der flüssigen Phase, vorzubeugen, können Stabilisatoren einzeln oder in Kombination zugegeben werden. Diese Stabilisatoren können aus folgenden Substanzgruppen stammen:
- oberflächenaktive metalloxidische Pulver mit Korngrößen im Nanometerbereich (5 bis 40 nm), z. B. SiO₂, Aerosile, TiO₂-P25, pyrogenes ZrO₂, Aluminiumoxid C
- geringe Mengen organischer Substanzen als Verdickungsmittel in Mengen von 0 bis 5 Gew.-%, bevorzugt in Men-gen von 0,05 bis 1 Gew.-%, zum Beispiel Zellulose, Me-thylcellulose und deren Derivate, Gelatine und/oder Alginate, und/oder
- Komplex- und/oder Chelatbildner wie Zitronensäure, Tween 20.

Zur Verbesserung der Haltbarkeit werden handelsübliche Konservierungsmittel, zum Beispiel Thiomersal, Rokonsal, Acticid SPX, Formaldehyd usw. zugegeben.

Es hat sich als vorteilhaft erwiesen, die Komponenten des Pastenopakers maschinell zu mischen, insbesondere zu homogenisieren, da dadurch eine gleichbleibende Qualität der Paste hinsichtlich der Homogenität und Viskosität und damit gleichbleibend sehr gute Auftrageigenschaften und Verarbeitungseigenschaften gegeben sind.

Die fertig angemischte Paste wird in eine handelsübliche Verpackung, insbesondere eine Glas- oder Plastikdose abgefüllt. Die Dose als Darreichungsform weist den Vorteil auf, daß bei eventuell aufgetretener Entmischung die Paste einfach vor Gebrauch mit einem Plastik- oder Glasspatel wieder aufgerührt werden kann. Die Entnahme der Paste mit dem Pinsel direkt aus der Dose hat den weiteren Vorteil, daß nur so viel Paste entnommen werden muß, wie unmittelbar gebraucht wird, was bei der Darreichung mittels einer Spritze nicht ohne weiteres gewährleistet ist.

Der Pastenopaker wird dann mit dem Pinsel auf das Metallgerüst der dentalen Restauration aufgetragen. Durch die sehr guten Auftrageigenschaften und die hervorragende Deckkraft des Pastenopakers ist das Applizieren von sehr dünnen Schichten auf das Metallgerüst möglich. Dadurch kann der Anwender platzsparend arbeiten und damit ästhetisch anspruchsvolle Arbeiten herstellen.

Das Brennen des Pastenopakers auf das Metallgerüst erfolgt im Temperaturbereich von 780°C bis 970°C. Die Brenntemperatur ist abhängig von der Zusammensetzung des Pulveropakers, der, wie oben erwähnt, aus Feldspatfritten unterschiedlicher Zusammensetzung bestehen kann und unterschiedliche physikalische Eigenschaften aufweisen kann.

Der erfindungsgemäße Pastenopaker, der aus der erfindungsgemäßen Pulvermischung herstellbar ist, weist gegenüber dem aus der EP 0 518 454 bekannten Pastenopaker mit hohem ZrO₂-Gehalt und dem aus der EP 0 280 985 bekannten opaken dentalkeramischen Material, das nur in Verbindung mit Streukristallen zu benutzen ist, folgende Vorteile auf:

Durch den Einsatz von Trübungsmitteln mit einer bestimmten Korngrößenverteilung und einer bestimmten spezifischen Oberfläche werden die Eigenschaften der Pulver-Flüssigkeitsmischung stark beeinflußt und gleichzeitig eine hervorragende Deckfähigkeit erreicht. Die Auftrageigenschaften des erfindungsgemäßen Opakers sind sehr gut, und selbst beim Auftragen sehr dünner Schichten wird eine hohe Deckkraft, auch ohne den Einsatz von Streukristallen, erreicht. Ein weiterer Vorteil des erfindungsgemäßen Opakers ist, daß dieser bei Brenntemperaturen unterhalb von 970°C auf das Metallgerüst aufgebrannt wird. Damit ist die Gefahr, daß sich das Gerüst auf-grund geringer Warmfestigkeit beim Aufbrennen der Keramik verzieht, eingeschränkt bzw. verringert. Die Oberfläche ist nach dem Brennen leicht rauh und weist einen seidenmatten Glanz auf. Diese Oberflächeneigenschaften sind von erheblichem Vorteil, da sich die nachfolgende Dentinschichtung gut auftragen läßt und der Verbund zwischen Opakerschicht und Dentinschicht verbessert wird. Als weiterer Vorteil wird die Darreichung in einem Glasgefäß gesehen.

Die Erfindung wird anhand der folgenden Beispiele näher erläutert.

### Beispiel 1

Ein Gemisch aus zerkleinertem Feldspat und mineralischen Oxiden wird bei hohen Temperaturen aufgeschmolzen und in Wasser abgeschreckt. Die so entstandene Feldspatfritte wird fein gemahlen und gegebenenfalls mit Fritten anderer chemischer Zusammensetzung gemischt. Die hergestellte Feldspatfritte, bestehend aus 55,6 Gew.-% SiO₂, 5,4 Gew.-% Na₂O, 1,4 Gew.-% CaO, 11,1 Gew.-% K₂O, 15,29 Gew.-% Al₂O₃, 8,33 Gew.-% TiO₂ und 2,88 Gew.-% B₂O₃ wird in einer Menge von 77 Gew.-Teilen mit 16 Gew.-Teilen CeO₂ und 7 Gew.-Teilen ZrO₂ gemischt. Es wird eine Mischung hergestellt aus 95 Teilen Butandiol-1.3 und 5 Teilen Silikonöl (Baysilon® -Öl M100). Diese Flüssigkeitsmischung wird in Mengen von 40 Teilen Flüssigkeit mit 60 Teilen Pulveropaker gemischt und in einem Universalmischer gemischt und homogenisiert. Es werden dazu 0,02 Gew.-Teile Methylhydroxypropylcellulose MHPC 8000 und 0,02 Gew.-Teile Zitronensäure zugefügt. Zur Erhöhung der Haltbarkeit werden als Konservierungsmittel 0,05 Gew.-Teile Thiomersal beigefügt.

Die Elementaranalyse der anorganischen Bestandteile des Pastenopakers ergibt folgende Zusammensetzung:
- SiO₂ 40,65%
- Na₂O 3,82%
- TiO₂ 5,74%
- CaO 1,03%
- K₂O 8,02%
- Al₂O₃ 11,18%
- ZrO₂ 6,71%
- SnO₂ < 0,01%
- CeO₂ 16,32%
- B₂O₃ 2,34%.

### Beispiel 2

80 Gew.-Teile der hergestellten Feldspatfritte der in Beispiel 1 genannten Zusammensetzung werden mit 20 Teilen SnO₂ gemischt. Die Flüssigkeit wird aus 65 Gew.-Teilen Butandiol-1.3, 9 Gew.-Teilen PEG 400, 24 Gew.-Teilen Glycerin und 2 Gew.-Teilen deionisiertem Wasser hergestellt. Die Flüssigkeitsmischung wird in Mengen von 50 Gew.-Teilen mit 50-Gew.-Teilen Pulveropaker in einem Universalmischer gemischt und homogenisiert. Um Entmischungen vorzubeugen, wird 1 Gew.-Teil Aerosil® R 972 zugegeben. Um die Haltbarkeit zu gewährleisten, werden 0,05 Gew.-Teile Acticid SPX zugegeben.

## Patentansprüche

1. Pulvermischung zur Herstellung eines Opakers enthaltend als Komponenten
- eine oder mehrere Feldspatfritten, die aus mehreren Feldspatfritten verschiedener chemischer Zusammensetzung und unterschiedlicher physikalischer Eigenschaften besteht oder bestehen, wobei die Feldspatfrtitte(n) eine maximale Korngräße unter 20 µm, einen d50 von 3 bis 6µm und einen d90 von 12 bis 16 µm aufweisen,
- CeO₂ als Trübungsmittel, gegebenenfalls weitere Trübungsmittel, insbesondere Oxide, wobei die als Trübungsmittel eingesetzten Oxide eine mittlere Korngröße von d50 < 1µm und eine maximale Korngröße < 2µm sowie eine spezifische Oberfläche von 1 bis 15m²/g aufweisen, sowie
- gegebenenfalls Pigmente.

2. Pulvermischung nach Anspruch 1, wobei die Trübungsmittel Metalloxide, wie SnO₂, TiO₂ und/oder ZrO₂ sind.

3. Pulvermischung nach mindestens einem der Ansprüche 1 bis 2, wobei die Pigmente farbige Metalloxide sind.

4. Pulvermischung nach mindestens einem der Ansprüche 1 bis 3, wobei die Mischung als Hauptkomponenten
- 30 bis 65 Gew.-% SiO₂,
- 2 bis 6 Gew.-% Na₂O,
- 3 bis 15 Gew.-% K₂O,
- 5 bis 15 Gew.-% Al₂O₃,
- 0,5 bis 10 Gew.-% CaO
- 0,5 bis 10 Gew.-% B₂O₃ sowie
- 0,5 bis 50 Gew.-% CeO₂
enthält.

5. Pulvermischung nach Anspruch 4, wobei als weitere Komponenten zusätzlich bis zu 50 Gew.-% TiO₂, 9 Gew.-% ZrO₂, sowie 50 Gew.-% SnO₂ in der Mischung enthalten sind.

6. Mischung in Pastenform enthaltend mindestens eine der Mischungen gemäß einem der Ansprüche 1 bis 5 und zusätzlich eine oder mehrere Flüssigkeiten zur Verleihung einer pastösen Konsistenz sowie gegebenenfalls Hilfsstoffe.

7. Mischung in Pastenform nach Anspruch 6, wobei die Flüs-sigkeiten Wasser, Modellierflüssigkeiten, zweiwertige Alkohole, Glycerin, Polyethylenglykole und/oder Silikonöle sind.

8. Mischung in Pastenform, wobei die Abmischungsverhältnisse von Flüssigkeit(en) zu trockener Mischung nach Anspruch 1 bis 5 zwischen 1 : 4 bis 1 : 1 liegen.

9. Mischung in Pastenform nach mindestens einem der Ansprüche 6 bis 8, wobei als Hilfsstoffe die Entmischung von Pulvermischung und Flüssigkeit verhindernde Stoffe Stabilisatoren und/oder Konservierungsmittel zugegeben sind.

10. Mischung in Pastenform nach Anspruch 9, wobei der oder die Stabilisator(en)
- oberflächenaktive metalloxidische Pulver mit Korngrößen im Nanometerbereich 5 bis 40 nm z. B. SiO₂, Aerosile, TiO₂-P25, pyrogenes ZrO₂, Aluminiumoxid C
- geringe Mengen organischer Substanzen als Verdickungsmittel in Mengen von 0 bis 5 Gew.-%, bevorzugt in Mengen von 0,05 bis 1 Gew.-%, zum Beispiel Zellulose, Methylcellulose und deren Derivate, Gelatine und/oder Alginate, und/oder
- Komplex- und/oder Chelatbildner wie Zitronensäure, Tween 20 sind.

## Claims

1. A powder composition for the preparation of an opaque matter, containing as components:
- one or more feldspar frits consisting of several feldspar frits of different chemical compositions and different physical properties, said feldspar frit(s) having a maximum grain size of below 20 µm, a d50 of from 3 to 6 µm and a d90 of from 12 to 16 µm;
- CeO₂ as an opacifier, optionally further opacifiers, especially oxides, the oxides employed as opacifiers having an average grain size of d50 < 1 µm and a maximum grain size of < 2 µm and a specific surface area of from 1 to 15 m²/g; and
- optionally pigments.

2. The powder composition according to claim 1, wherein said opacifiers are metal oxides, such as SnO₂, TiO₂ and/or ZrO₂.

3. The powder composition according to at least one of claims 1 to 2, wherein said pigments are colored metal oxides.

4. The powder composition according to at least one of claims 1 to 3, wherein said composition contains, as its main components:
- from 30 to 65% by weight of SiO₂;
- from 2 to 6% by weight of Na₂O;
- from 3 to 15% by weight of K₂O;
- from 5 to 15% by weight of Al₂O₃;
- from 0.5 to 10% by weight of CaO;
- from 0.5 to 10% by weight of B₂O₃; and
- from 0.5 to 50% by weight of CeO₂.

5. The powder composition according to claim 4, wherein up to 50% by weight of TiO₂, 9% by weight of ZrO₂ and 50% by weight of SnO₂ are additionally contained in the composition as further components.

6. A composition in a paste form containing at least one of the compositions according to any of claims 1 to 5 and additionally one or more liquids for providing a paste-like consistency, and optionally auxiliary agents.

7. The composition in a paste form according to claim 6, wherein said liquids are water, modeling liquids, dihydric alcohols, glycerol, polyethylene glycols and/or silicone oils.

8. A composition in a paste form wherein the mixing ratios of liquid(s) to the dry composition according to claims 1 to 5 are between 1:4 and 1:1.

9. The composition in a paste form according to at least one of claims 6 to 8, wherein substances preventing the segregation of the powder composition and liquid, stabilizers and/or preservatives are added as said auxiliary agents.

10. The composition in a paste form according to claim 9, wherein said stabilizers are:
- surface-active metal-oxide powders having grain sizes in the nanometer range, from 5 to 40 nm, e.g., SiO₂, aerosils, TiO₂-P25, pyrogenic ZrO₂, alumina C;
- small amounts of organic substances as thickeners in quantities of from 0 to 5% by weight, preferably in quantities of from 0.05 to 1% by weight, for example, cellulose, methylcellulose and their derivatives, gelatin and/or alginates; and/or
- complexing and/or chelating agents, such as citric acid, Tween 20.

## Revendications

1. Mélange de poudres pour préparer un opacifiant, contenant en tant que composants :
- une ou plusieurs frittes de feldspath, qui est ou sont constituées de plusieurs frittes de feldspath ayant différentes compositions chimiques et différentes propriétés physiques, la ou les frittes de feldspath ayant une granulométrie maximale inférieure à 20 *µ*m, un d50 de 3 à 6 *µ*m et un d90 de 12 à 16 *µ*m,
- du CeO₂ en tant qu'agent de turbidité, éventuellement d'autres agents de turbidité, notamment des oxydes, les oxydes utilisés en tant qu'agents de turbidité ayant une granulométrie moyenne d50 < 1 *µ*m et une granulométrie maximale < 2 *µ*m, ainsi qu'une aire spécifique de 1 à 15 m²/g, et
- éventuellement des pigments.

2. Mélange de poudres selon la revendication 1, dans lequel les agents de turbidité sont des oxydes métalliques tels que SnO₂, TiO₂ et/ou ZrO₂.

3. Mélange de poudres selon au moins l'une des revendications 1 et 2, dans lequel les pigments sont des oxydes métalliques colorés.

4. Mélange de poudres selon au moins l'une des revendications 1 à 3, dans lequel le mélange contient en tant que composants principaux
- 30 à 65 % en poids de SiO₂,
- 2 à 6 % en poids de Na₂O,
- 3 à 15 % en poids de K₂O,
- 5 à 15 % en poids d'Al₂O₃,
- 0,5 à 10 % en poids de CaO,
- 0,5 à 10 % en poids de B₂O₃, ainsi que
- 0,5 à 50 % en poids de CeO₂.

5. Mélange de poudres selon la revendication 4, dans lequel le mélange contient en outre, en tant qu'autres composants, jusqu'à 50 % en poids de TiO₂, jusqu'à 9 % en poids de ZrO₂, ainsi que jusqu'à 50 % en poids de SnO₂.

6. Mélange sous forme de pâte contenant au moins l'un des mélanges selon l'une des revendications 1 à 5, et en outre un ou plusieurs liquides destinés à conférer une consistance pâteuse, et éventuellement des adjuvants.

7. Mélange sous forme de pâte selon la revendication 6, dans lequel les liquides sont de l'eau, des liquides à modeler, des dialcools, de la glycérine, des polyéthylèneglycols et/ou les huiles de silicone.

8. Mélange sous forme de pâte, dans lequel les rapports de mélange du ou des liquides au mélange sec selon les revendications 1 à 5 sont compris entre 1:4 et 1:1.

9. Mélange sous forme de pâte selon au moins l'une des revendications 6 à 8, dans lequel on ajoute en tant qu'adjuvants des substances (stabilisants) empêchant la démixtion du mélange de poudres et du liquide, et/ou des conservateurs.

10. Mélange sous forme de pâte selon la revendication 9, dans lequel le ou les stabilisants sont
- des poudres tensioactives à base d'oxydes métalliques ayant une granulométrie dans la plage nanométrique de 6 à 40 nm, par exemple le SiO₂, les Aerosils, le TiO₂-P25, le ZrO₂ pyrogène, l'oxyde d'aluminium C,
- de petites quantités de substances organiques servant d'épaississants dans des quantités de 0 à 5 % en poids, de préférence dans des quantités de 0,05 à 1 % en poids, par exemple la cellulose, la méthylcellulose et ses dérivés, la gélatine et/ou les alginates, et/ou
- des complexants et/ou chélatants, tels que l'acide citrique, le Tween 20.
